# EUROPEAN PATENT SPECIFICATION

(11) **EP 4 291 328 B1**
(45) Date of publication and mention of the grant of the patent: **23.09.2026**
(21) Application number: 22753314.8
(22) Date of filing: 10.02.2022
(51) Int. Cl.: C07F 15/00, C07C 67/343, C07C 67/347, B01J 23/46, B01J 31/22

(54) **DIRUTHENIUM CATALYST COMPOSITIONS AND SYNTHETIC PROCESSES RELATED THERETO**
DIARUTHENIUMKATALYSATORZUSAMMENSETZUNGEN UND DAMIT VERBUNDENE SYNTHETISCHE VERFAHREN
COMPOSITIONS DE CATALYSEUR DE DIRUTHÉNIUM ET PROCÉDÉS DE SYNTHÈSE S'Y RAPPORTANT

(30) Priority: 10.02.2021 US 202163147800 P
(43) Date of publication of application: 20.12.2023
(73) Proprietor: Emory University, Atlanta, Georgia 30322 (US)
(72) Inventor: DAVIES, Huw M. L., Atlanta, Georgia 30322 (US); SAILER, Joshua, Atlanta, Georgia 30322 (US); SHARLAND, Jack C., Atlanta, Georgia 30322 (US); WEI, Bo, Atlanta, Georgia 30322 (US)
(74) Representative: Coles, Andrea Birgit
(86) International application number: PCT/US2022/015912
(87) International publication number: WO 2022/173899

(56) References cited:
- WO-A2-2012/167198
- WO-A2-2012/167198
- JP-B1- S4 928 489
- US-A1- 2014 323 767
- US-B1- 6 410 746
- DATABASE CAPLUS [online] 1 January 2015 (2015-01-01), SU YAN: "Diastereo-and enantioselective cyclopropanation of alkenyl fluorides with benzyl diazoarylacetates", XP093241925, Database accession no. 2015:309947
- SU YAN ET AL: "Diastereo- and enantioselective cyclopropanation of alkyenyl fluorides with benzyl diazoarylacetates", TETRAHEDRON LETTERS, vol. 56, no. 14, 1 April 2015 (2015-04-01), AMSTERDAM, NL, pages 1805 - 1807, XP093241924, ISSN: 0040-4039, DOI: 10.1016/j.tetlet.2015.02.044

## Description

### BACKGROUND

The cyclopropane ring is a common structural motif incorporated into many pharmaceutical agents. Elaborate chiral cyclopropanes have been incorporated into therapeutic scaffolds, such as the trisubstituted cyclopropanes in beclabuvir, paritaprevir, and glecaprevir. In these cases, three substituents are placed in a defined orientation. The syntheses of these cyclopropanes are challenging because they contain two stereogenic centers which are to be generated in a diastereoselective and enantioselective manner. Thus, there is a need to identify improved synthetic processes.

Davies et al. report asymmetric cyclopropanations by rhodium(II) N-(arylsulfonyl)prolinate catalyzed decomposition of vinyldiazomethanes in the presence of alkenes. J. Am. Chem. Soc. 1996, 118, 6897-6907.

Saha et al. report evaluation of a pair of diruthenium(I) catalysts for carbene-transfer reactions from ethyl diazoacetate. Organometallics, 2011, 30, 2051-2058.

Chepiga et al. report a guide to enantioselective dirhodium(II)-catalyzed cyclopropanation with aryldiazoacetates. Tetrahedron, 2013, 69, 5765-5771. See also US Patent No. 8,975,428.

Miyazawa et al. report chiral paddle-wheel diruthenium complexes for asymmetric catalysis. Nature Catalysis, 2020, 3, 851-858.

Su Yan et al. report rhodium complexes and their use for enantioselective cyclopropanation of alkenyl fluorides with benzyl diazoarylacetates. Tetrahedron Letters, vol. 56, no 14, 1805-1807.

JPS4928489B1 relates to a diruthenium moiety and a method for producing acrylonitrile dimer.

WO 2012/167198 relates to compositions comprising dirhodium catalysts and their use in processes such as enantioselective transformations of donor/acceptor carbenoids.

References cited herein are not an admission of prior art.

### SUMMARY

This disclosure relates to compositions comprising diruthenium catalysts and uses related thereto. In certain embodiments, the diruthenium catalyst comprises a cyclopropyl ring substituted with a carboxylic acid ligand. In certain embodiments, the diruthenium catalyst comprises an N-(sulfonyl)pyrrolidine ring substituted with a carboxylic acid ligand. In certain embodiments, the diruthenium catalyst comprises a 2-(1,3-dioxoisoindolin-2-yl)acetic acid ligand. In certain embodiments, this disclosure relates to methods of using catalysts in chemical transformations disclosed herein.

The present invention provides a composition comprising a diruthenium complex of the following formula, 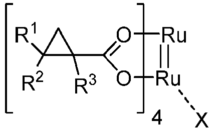
or salt thereof, wherein
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

The present invention also provides a composition comprising a diruthenium complex of the following formula, 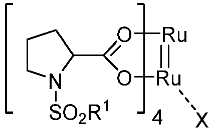
or salt thereof, wherein
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

The present invention also provides a composition comprising a diruthenium complex of the following formula, 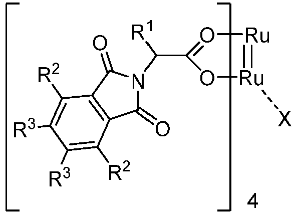
or salt thereof, wherein
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the disclosure relates to compositions comprising a catalyst of the following formula, 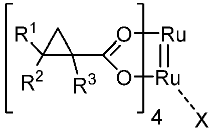 or derivatives or salts thereof wherein, R¹, R², R³ and X are defined herein.

In certain embodiments, the disclosure relates to compositions comprising Ru₂(*S*-p-Ph-TPCP)₄ X , Ru₂(*S*-p-Br-TPCP)₄X, Ru₂(*S*-2Cl-5Br-TPCP)₄X, Ru₂(*R*-tris(p-^{t}BuC₆H₄)-TPCP)₄X, or a catalyst of formula 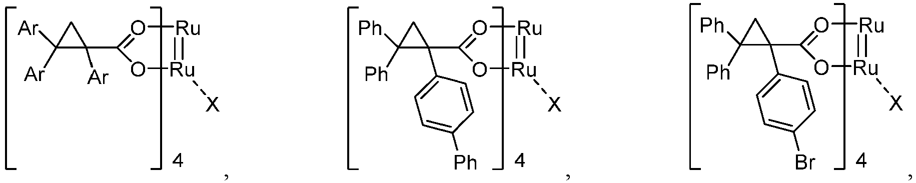 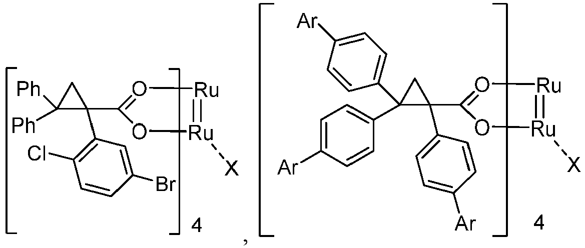 or derivatives or salts thereof wherein, Ar and X are defined herein, e.g., X is a covalently bound ligand, such as Cl, or a counterion such as PF₅⁻ or BARF⁻.

In certain embodiments, the disclosure relates to compositions comprising Ru₂(*R-*DOSP)₄ X , or a catalyst of formula 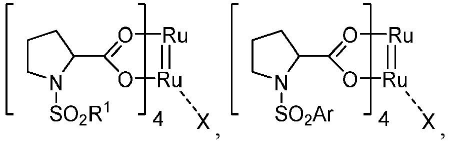 or derivatives or salts thereof wherein, R¹ and X are defined herein, e.g., X is a covalently bound ligand, such as Cl, or a counterion such as PF₅⁻ or BARF⁻.

In certain embodiments, Ar is p-alkyl-C₆H₄-.

In certain embodiments, the disclosure relates to compositions comprising Ru₂(*S-*TCPTTL)₄ X , Ru₂(*S*-PTAD)₄ X, Ru₂(*S*-TCPTAD)₄ X or a catalyst of formula 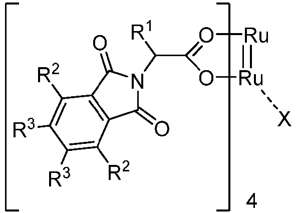 or derivatives or salts thereof wherein, R¹, R², R³, and X are defined herein.

In certain embodiments, the disclosure relates to methods of preparing diruthenium catalysts or intermediates disclosed herein by mixing starting materials with catalysts under conditions such that the catalysts or intermediates are formed.

In certain embodiments, the disclosure contemplates enantioselective reactions of donor/acceptor carbenoids, such as cyclopropanations, formal [4 + 3] cycloadditions, C-H functionalizations, and ylide transformations comprising mixing a compound comprising a carbenoid precursor, e.g., a diazo compound, and catalysts disclosed herein and reactive compounds under conditions such that a synthetic compound is formed.

### BRIEF DESCRIPTION OF THE DRAWINGS

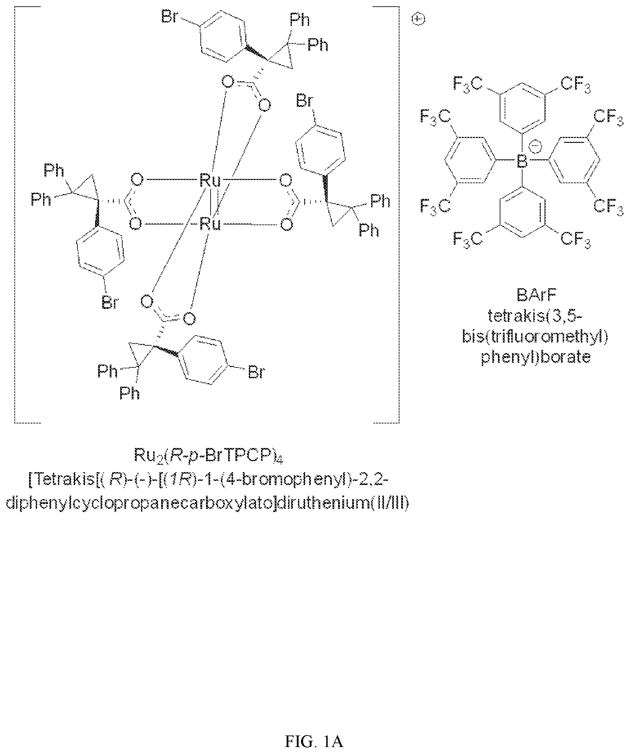

Figure 1A illustrates the proposed chemical structure of Ru₂(R-p-BrTPCP)₄; [Tetrakis[(R)-(-)-[(1R)-1-(4-bromophenyl)-2,2-diphenylcyclopropanecarboxylato] diruthenium(II/III) with a tetrakis(3,5-bis(trifluoromethyl)phenyl)borate (BArF) counter anion.

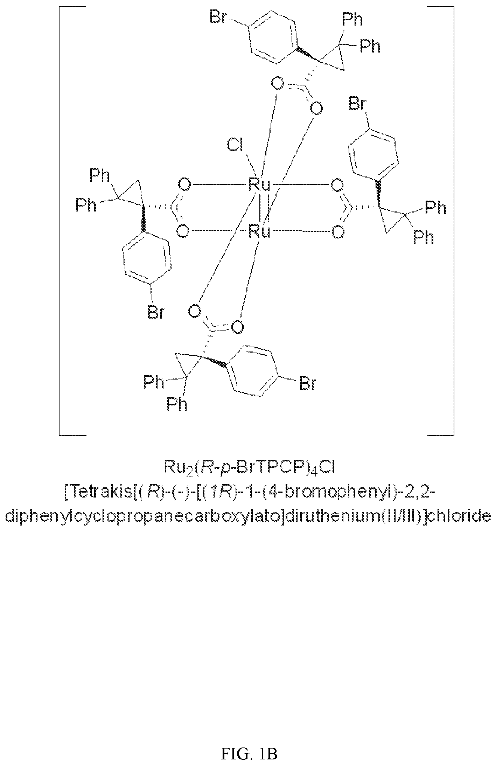

Figure 1B illustrates the proposed chemical structure of Ru₂(R-p-BrTPCP)₄Cl; [Tetrakis[(R)-(-)-[(1R)-1-(4-bromophenyl)-2,2-diphenylcyclopropanecarboxylato] diruthenium(II/III)]chloride.

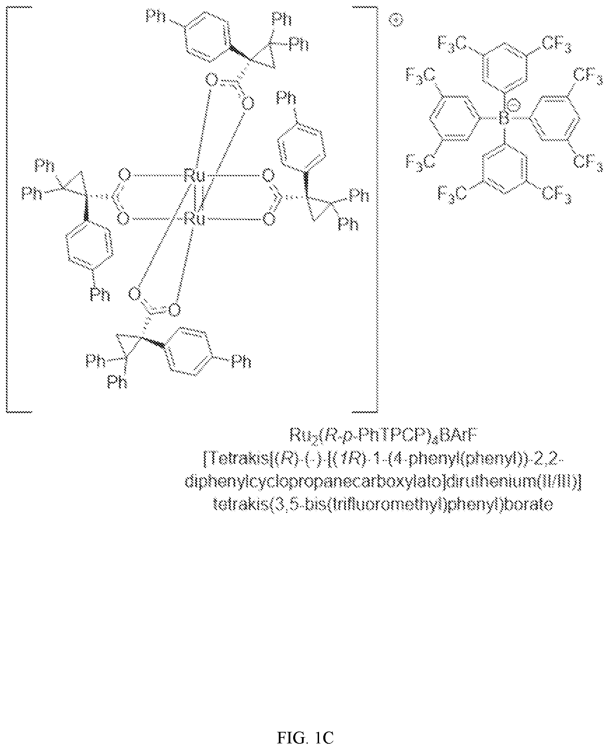

Figure 1C illustrates the proposed chemical structure of Ru₂(R-p-PhTPCP)₄BArF; [Tetrakis[(R)-(-)-[(1R)-1-(4-phenyl(phenyl))-2,2-diphenylcyclopropanecarboxylato] diruthenium (II/III)] with a tetrakis(3,5-bis(trifluoromethyl)phenyl)borate counter anion.

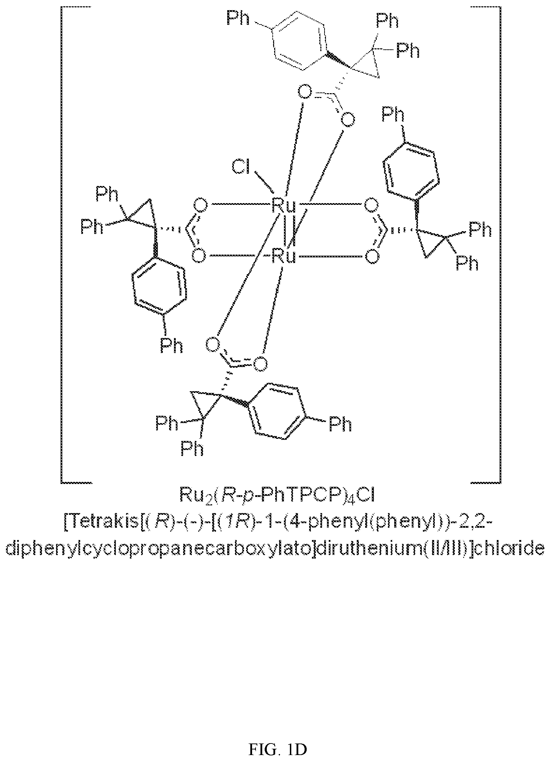

Figure 1D illustrates the proposed chemical structure of Ru₂(R-p-PhTPCP)₄Cl; [Tetrakis[(R)-(-)-[(1R)-1-(4-phenyl(phenyl))-2,2-diphenylcyclopropanecarboxylato] diruthenium(II/III)]chloride.

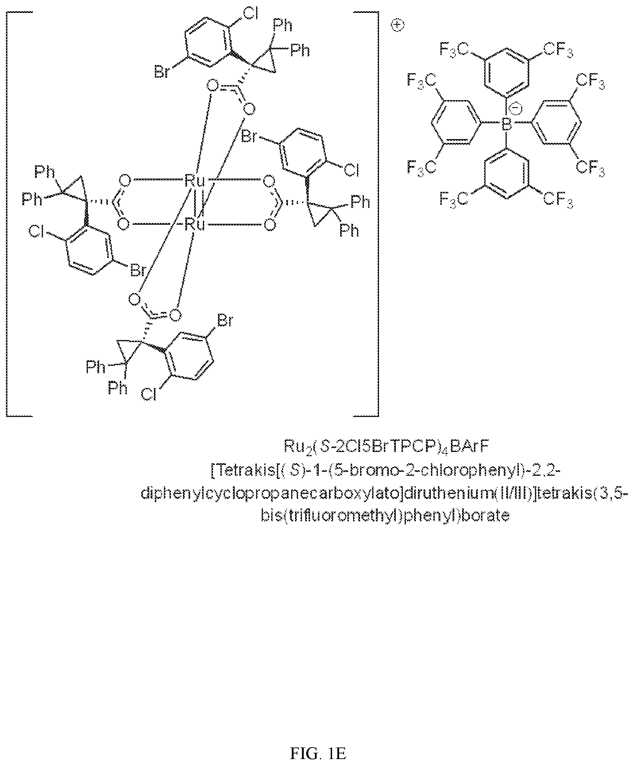

Figure 1E illustrates the proposed chemical structure of Ru₂(S-2Cl5BrTPCP)₄BArF; [Tetrakis[(S)-1-(5-bromo-2-chlorophenyl)-2,2-diphenylcyclopropanecarboxylato] diruthenium(II/III)] with a tetrakis(3,5-bis(trifluoromethyl)phenyl)borate counter anion.

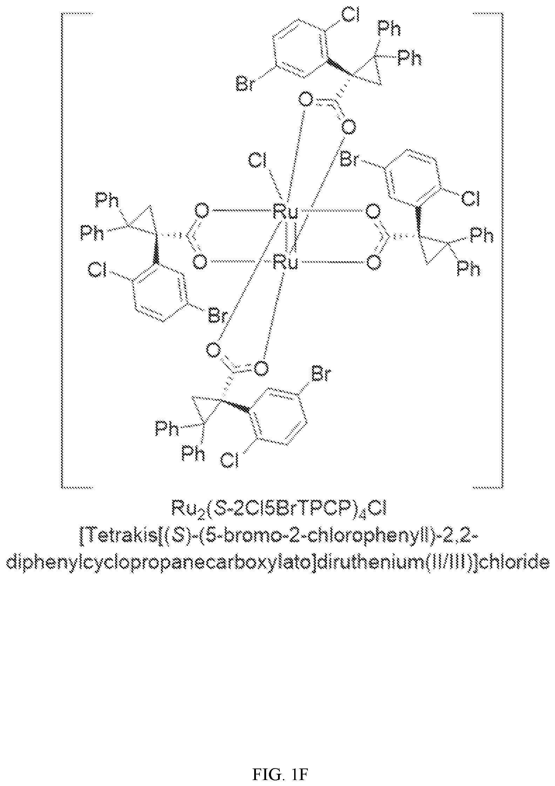

Figure 1F illustrates the proposed chemical structure of Ru₂(S-2Cl5BrTPCP)₄Cl; [Tetrakis[(S)-(5-bromo-2-chlorophenyll)-2,2-diphenylcyclopropanecarboxylato] diruthenium(II/III)]chloride.

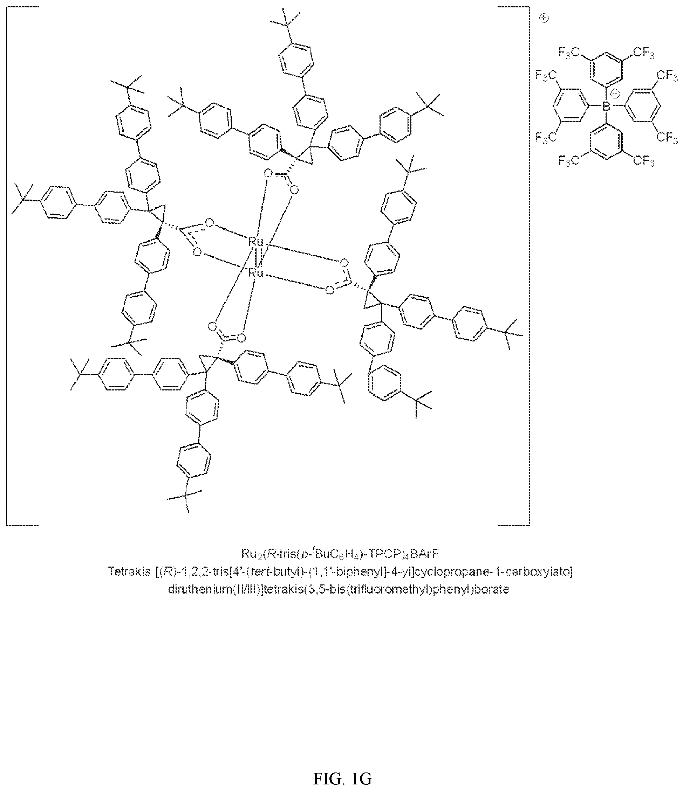

Figure 1G illustrates the proposed chemical structure of Ru₂(R-tris(p-tBuC₆H₄)-TPCP)₄BArF; Tetrakis [(R)-1,2,2-tris[4'-(tert-butyl)-(1,1'-biphenyl]-4-yl]cyclopropane-1-carboxylato] diruthenium(II/III)] with a tetrakis(3,5-bis(trifluoromethyl)phenyl)borate counter anion.

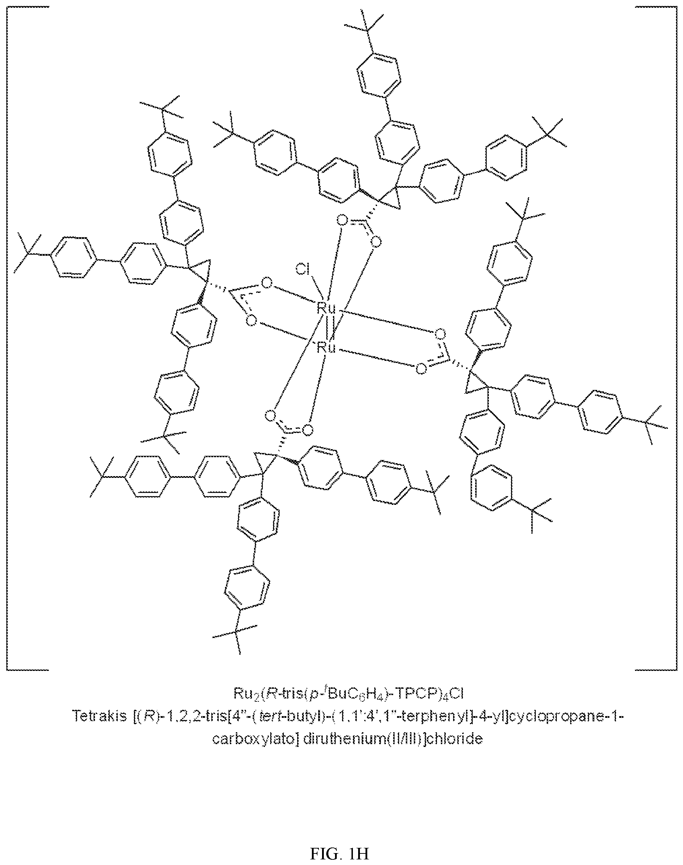

Figure 1H illustrates the proposed chemical structure of Ru₂(R-tris(p-tBuC₆H₄)-TPCP)₄Cl; Tetrakis [(R)-1,2,2-tris[4"-(tert-butyl)-(1,1':4',1"-terphenyl]-4-yl]cyclopropane-1-carboxylato] diruthenium(II/III)]chloride.

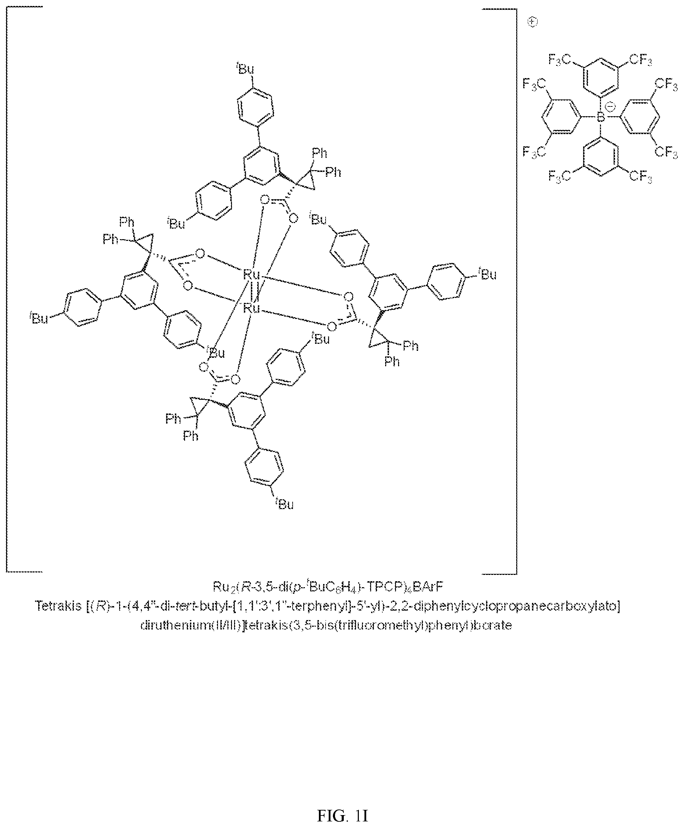

Figure 1I illustrates the proposed chemical structure of Ru₂(R-3,5-di(p-tBuC₆H₄)-TPCP)₄BArF; Tetrakis [(R)-1-(4,4"-di-tert-butyl-[1,1':3',1"-terphenyl]-5'-yl)-2,2-diphenylcyclopropanecarboxylato] diruthenium(II/III)] with a tetrakis(3,5-bis(trifluoromethyl)phenyl)borate counter anion.

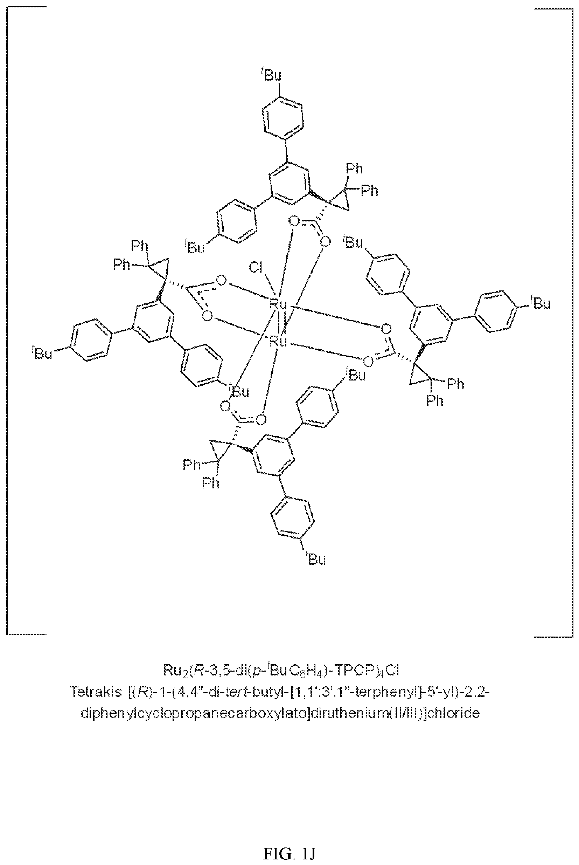

Figure 1J illustrates the proposed chemical structure of Ru₂(R-3,5-di(p-tBuC₆H₄)-TPCP)₄Cl; Tetrakis [(R)-1-(4,4"-di-tert-butyl-[1,1':3',1"-terphenyl]-5'-yl)-2,2-diphenyl cyclopropanecarboxylato]diruthenium(II/III)]chloride.

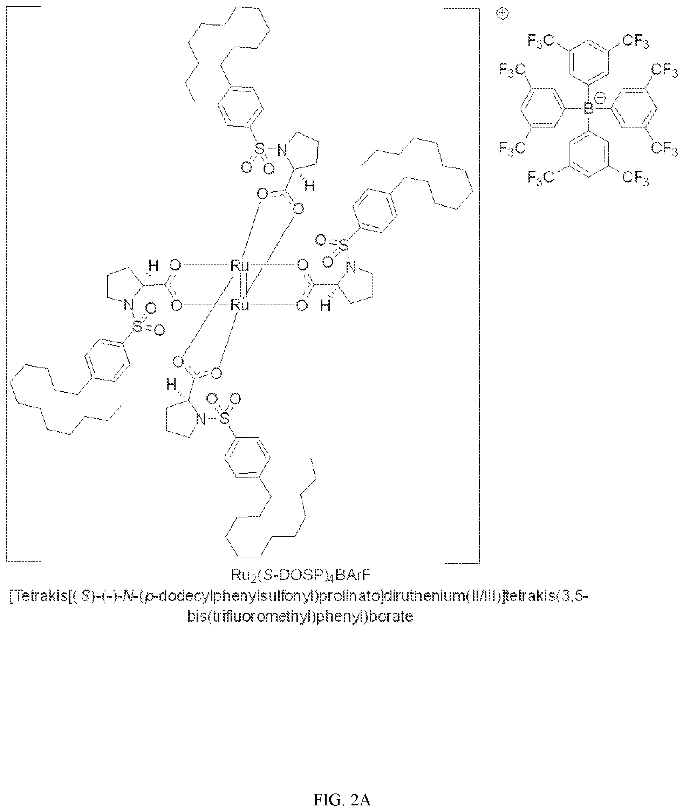

Figure 2A illustrates the proposed chemical structure of Ru₂(S-DOSP)₄BArF; [Tetrakis[(S)-(-)-N-(p-dodecylphenylsulfonyl)prolinato]diruthenium(II/III)] with a tetrakis(3,5-bis(trifluoromethyl)phenyl)borate counter anion.

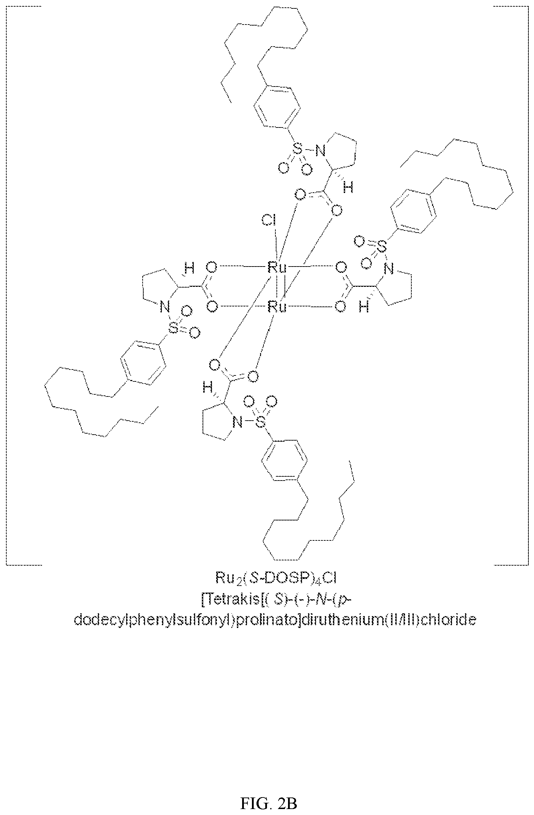

Figure 2B illustrates the proposed chemical structure of Ru₂(S-DOSP)₄Cl; [Tetrakis[(S)-(-)-N-(p-dodecylphenylsulfonyl)prolinato]diruthenium(II/III)chloride.
Figure 3A illustrates the proposed chemical structure of Ru₂(S-TPPTTL)₄BArF [Tetrakis[N-tetraphenylphthaloyl-(S)-tert-leucinato]diruthenium(II/III)] with a tetrakis(3,5-bis(trifluoromethyl)phenyl)borate counter anion.
Figure 3B illustrates the proposed chemical structure of Ru₂(S-TPPTTL)₄Cl; [Tetrakis[N-tetraphenylphthaloyl-(S)-tert-leucinato]diruthenium(II/III)]chloride.
Figure 3C illustrates the proposed chemical structure of Ru₂(S-TCPTAD)₄BArF; [Tetrakis[(S)-(+)-(1-adamantyl)-(N-tetrachlorophthalimido)acetato]diruthenium(II/III)] with a tetrakis(3,5-bis(trifluoromethyl)phenyl)borate counter anion.
Figure 3D illustrates the proposed chemical structure of Ru₂(S-TCPTAD)₄Cl; [Tetrakis[(S)-(+)-(1-adamantyl)-(N-tetrachlorophthalimido)acetato]diruthenium(II/III)] chloride.
Figure 3E illustrates the proposed chemical structure of Ru₂(S-PTAD)₄BArF; [Tetrakis[(S)-(+)-(1-adamantyl)-(N-phthalimido)acetato]diruthenium(II/III)] with a tetrakis(3,5-bis(trifluoromethyl)phenyl)borate counter anion.
Figure 3F illustrates the proposed chemical structure of Ru₂(S-PTAD)₄Cl; [Tetrakis[(S)-(+)-(1-adamantyl)-(N-phthalimido)acetato]diruthenium(II/III)]chloride.
Figure 4 illustrates the use of diruthenium complexes disclosed herein in the synthesis of intermediates for the production pharmaceutical products such as beclabuvir.

### DETAILED DESCRIPTION

### Terms

Unless defined otherwise, all technical and scientific terms used herein have the same meaning as is commonly understood by one of skill in the art. The following definitions are provided to help interpret the disclosure and claims of this application. In the event a definition in this section is not consistent with definitions elsewhere, the definition set forth in this section will control.

As used herein but not claimed, the term "derivative" refers to a structurally similar compound that retains sufficient functional attributes of the identified analogue. The derivative may be structurally similar because it is lacking one or more atoms, substituted, a salt, in different hydration/oxidation states, or because one or more atoms within the molecule are switched, such as, but not limited to, replacing an oxygen atom with a sulfur atom or replacing an amino group with a hydroxy group, or visa versa. Derivatives may be prepared by any variety of synthetic methods or appropriate adaptations presented in synthetic or organic chemistry textbooks, such as those provide in March's Advanced Organic Chemistry: Reactions, Mechanisms, and Structure, Wiley, 6th Edition (2007) Michael B. Smith or Domino Reactions in Organic Synthesis, Wiley (2006) Lutz F. Tietze.

The term "substituted" refers to a molecule wherein at least one hydrogen atom is replaced with a substituent. When substituted, one or more of the groups are "substituents." The molecule may be multiply substituted. In the case of an oxo substituent ("=O"), two hydrogen atoms are replaced. Example substituents within this context may include halogen, hydroxy, alkyl, alkoxy, nitro, cyano, oxo, carbocyclyl, carbocycloalkyl, heterocarbocyclyl, heterocarbocycloalkyl, aryl, arylalkyl, heteroaryl, heteroarylalkyl, -NRaRb, -NRaC(=O)Rb, -NRaC(=O)NRaNRb, -NRaC(=O)ORb, - NRaSO2Rb, -C(=O)Ra, -C(=O)ORa, -C(=O)NRaRb, -OC(=O)NRaRb, -ORa, -SRa, -SORa, -S(=O)2Ra, -OS(=O)2Ra and -S(=O)2ORa. Ra and Rb in this context may be the same or different and independently hydrogen, halogen hydroxy, alkyl, alkoxy, alkyl, amino, alkylamino, dialkylamino, carbocyclyl, carbocycloalkyl, heterocarbocyclyl, heterocarbocycloalkyl, aryl, arylalkyl, heteroaryl, heteroarylalkyl.

When used in reference to compound(s) disclosed herein, "salts" refer to derivatives of the disclosed compound(s) where the parent compound is modified making acid or base salts thereof. Examples of salts include, but are not limited to, mineral or organic acid salts of basic residues such as amines, alkylamines, or dialkylamines; alkali or organic salts of acidic residues such as carboxylic acids; and the like.

As used herein, "alkyl" means a noncyclic straight chain or branched saturated hydrocarbon such as those containing from 1 to 10 carbon atoms. Representative saturated straight chain alkyls include methyl, ethyl, n-propyl, n-butyl, n-pentyl, n-hexyl, n-septyl, n-octyl, n-nonyl, and the like; while saturated branched alkyls include isopropyl, sec-butyl, isobutyl, tert-butyl, isopentyl, and the like. Unsaturated alkyls contain at least one double or triple bond between adjacent carbon atoms (referred to as an "alkenyl" or "alkynyl", respectively). Representative straight chain and branched alkenyls include ethylenyl, propylenyl, 1-butenyl, 2-butenyl, isobutylenyl, 1-pentenyl, 2-pentenyl, 3 -methyl-1-butenyl, 2-methyl-2-butenyl, 2,3- dimethyl-2-butenyl, and the like; while representative straight chain and branched alkynyls include acetylenyl, propynyl, 1-butynyl, 2-butynyl, 1-pentynyl, 2-pentynyl, 3- methyl-1-butynyl, and the like.

Non-aromatic mono or polycyclic alkyls are referred to herein as "carbocycles" or "carbocyclyl" groups. Representative saturated carbocycles include cyclopropyl, cyclobutyl, cyclopentyl, cyclohexyl, and the like; while unsaturated carbocycles include cyclopentenyl and cyclohexenyl, and the like.

"Heterocarbocycles" or heterocarbocyclyl" groups are carbocycles which contain from 1 to 4 heteroatoms independently selected from nitrogen, oxygen and sulfur which may be saturated or unsaturated (but not aromatic), monocyclic or polycyclic, and wherein the nitrogen and sulfur heteroatoms may be optionally oxidized, and the nitrogen heteroatom may be optionally quaternized. Heterocarbocycles include morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

"Aryl" or "Ar" means an aromatic carbocyclic monocyclic or polycyclic ring such as phenyl or naphthyl. Polycyclic ring systems may, but are not required to, contain one or more non-aromatic rings, as long as one of the rings is aromatic.

As used herein, "heteroaryl" or "heteroaromatic" refers an aromatic heterocarbocycle having 1 to 4 heteroatoms selected from nitrogen, oxygen and sulfur, and containing at least 1 carbon atom, including both mono- and polycyclic ring systems. Polycyclic ring systems may, but are not required to, contain one or more non-aromatic rings, as long as one of the rings is aromatic. Representative heteroaryls are furyl, benzofuranyl, thiophenyl, benzothiophenyl, pyrrolyl, indolyl, isoindolyl, azaindolyl, pyridyl, quinolinyl, isoquinolinyl, oxazolyl, isooxazolyl, benzoxazolyl, pyrazolyl, imidazolyl, benzimidazolyl, thiazolyl, benzothiazolyl, isothiazolyl, pyridazinyl, pyrimidinyl, pyrazinyl, triazinyl, cinnolinyl, phthalazinyl, and quinazolinyl. It is contemplated that the use of the term "heteroaryl" includes N-alkylated derivatives such as a 1-methylimidazol-5-yl substituent.

As used herein, "heterocycle" or "heterocyclyl" refers to mono- and polycyclic ring systems having 1 to 4 heteroatoms selected from nitrogen, oxygen and sulfur, and containing at least 1 carbon atom. The mono- and polycyclic ring systems may be aromatic, non-aromatic or mixtures of aromatic and non-aromatic rings. Heterocycle includes heterocarbocycles, heteroaryls, and the like.

"Alkylthio" refers to an alkyl group as defined above attached through a sulfur bridge. An example of an alkylthio is methylthio, (i.e., -S-CH₃).

"Alkoxy" refers to an alkyl group as defined above attached through an oxygen bridge. Examples of alkoxy include, but are not limited to, methoxy, ethoxy, n-propoxy, i-propoxy, n-butoxy, s-butoxy, t-butoxy, n- pentoxy, and s-pentoxy. Preferred alkoxy groups are methoxy, ethoxy, n-propoxy, i- propoxy, n-butoxy, s-butoxy, t-butoxy.

"Alkylamino" refers an alkyl group as defined above attached through an amino bridge. An example of an alkylamino is methylamino, (i.e., -NH-CH₃).

"Alkanoyl" refers to an alkyl as defined above attached through a carbonyl bridge (i.e., -(C=O)alkyl).

"Alkylsulfonyl" refers to an alkyl as defined above attached through a sulfonyl bridge (i.e., -S(=O)₂alkyl) such as mesyl and the like, and "Arylsulfonyl" refers to an aryl attached through a sulfonyl bridge (i.e., - S(=O)₂aryl).

"Alkylsulfamoyl" refers to an alkyl as defined above attached through a sulfamoyl bridge (i.e., -S(=O)₂NHalkyl), and an "Arylsulfamoyl" refers to an alkyl attached through a sulfamoyl bridge (i.e., - S(=O)₂NHaryl).

"Alkylsulfinyl" refers to an alkyl as defined above with the indicated number of carbon atoms attached through a sulfinyl bridge (i.e. -S(=O)alkyl).

The terms "halogen" and "halo" refer to fluorine, chlorine, bromine, and iodine.

### Diruthenium catalysts

In certain embodiments, the disclosure relates to a diruthenium catalyst comprising a cyclopropyl ring substituted with a carboxylic acid ligand.

In certain embodiments, the disclosure relates to compositions comprising a diruthenium complex of the following formula, 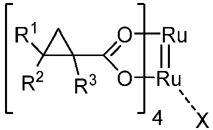 or salts thereof wherein,
X is a covalently bound ligand, such as Cl or other halogen, or a counterion such as PF₅⁻ or BARF⁻;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the disclosure relates to compositions comprising a diruthenium complex of the following formula, 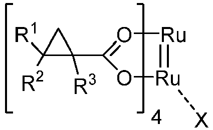 salt thereof wherein,
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the counter anion is tetrakis(3,5-bis(trifluoromethyl)phenyl)borate (BArF).

In certain embodiments, the ligand is a halogen or chlorine (Cl).

In certain embodiments, the diruthenium complex is tetrakis [1-(4-phenyl(phenyl))-2,2-diphenylcyclopropanecarboxylato]diruthenium.

In certain embodiments, the diruthenium complex is tetrakis[1-(5-bromo-2-chlorophenyl)-2,2-diphenylcyclopropanecarboxylato]diruthenium.

In certain embodiments, the diruthenium complex is tetrakis [1,2,2-tris[4'-(tert-butyl)-(1,1'-biphenyl]-4-yl]cyclopropane-1-carboxylato] diruthenium].

In certain embodiments, the diruthenium complex is tetrakis [1-(4,4"-di-tert-butyl-[1,1':3',1"-terphenyl]-5'-yl)-2,2-diphenylcyclopropanecarboxylato] diruthenium.

In certain embodiments, the diruthenium complex is tetrakis[1-(4-bromophenyl)-2,2-diphenylcyclopropanecarboxylato]diruthenium.

In certain embodiments, the diruthenium complex is in a composition with greater than 55%, 75%, 85%, 95%, 98%, or 99% enantiomeric excess of the following stereoisomers:

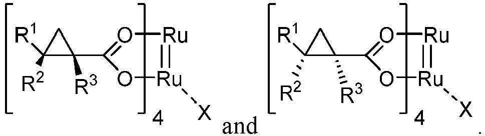

In certain embodiments, R¹ is carbocyclyl, aryl, or heterocyclyl.

In certain embodiments, R¹ is an aryl or an aromatic heterocyclyl.

In certain embodiments, R¹ is a phenyl optionally substituted with one or more R⁴.

In certain embodiments, R¹ is a phenyl optionally substituted with one or more halogen, alkyl, or alkoxy.

In certain embodiments, R¹ is hydrogen.

In certain embodiments, R² is carbocyclyl, aryl, or heterocyclyl.

In certain embodiments, R² is an aryl or an aromatic heterocyclyl.

In certain embodiments, R² is a phenyl optionally substituted with one or more R⁴.

In certain embodiments, R² is a phenyl optionally substituted with one or more halogen, alkyl, or alkoxy.

In certain embodiments, R³ is carbocyclyl, aryl, or heterocyclyl.

In certain embodiments, R³ is an aryl or an aromatic heterocyclyl.

In certain embodiments, R³ is a phenyl optionally substituted with one or more R⁴.

In certain embodiments, R³ is a phenyl optionally substituted with one or more halogen, alkyl, or alkoxy.

In certain embodiments, R³ is a phenyl substituted with a halogen.

In certain embodiments, R³ is a phenyl substituted with a halogen in the otho or para position.

In certain embodiments, the disclosure relates to compositions comprising a diruthenium complex of the following formula, 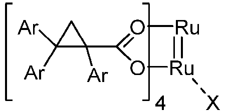 wherein X is a covalently bound ligand, such as a halogen, Cl, or a counterion such as PF₅⁻ or BARF⁻.

In certain embodiments, the disclosure relates to compositions comprising a diruthenium complex of the following formula, 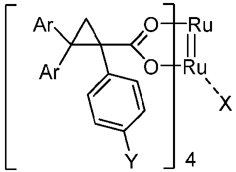
wherein X is a covalently bound ligand, such as a halogen, Cl, or a counterion such as PF₅⁻ or BARF⁻ and
Y is an electron withdrawing group.

In certain embodiments, the catalyst is in a composition with greater than 55%, 75%, 85%, 95%, 98%, or 99% enantiomeric excess of the following stereoisomers:

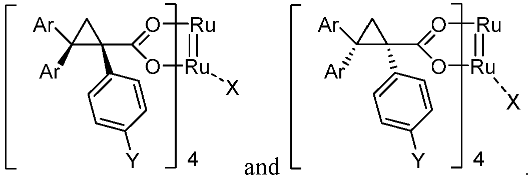

In certain embodiments, Y is a halogen.

In certain embodiments, the disclosure relates to compositions comprising a diruthenium complex of the following formula, 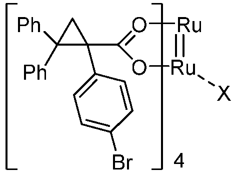 wherein X is a covalently bound ligand, such as a halogen, Cl, or a counterion such as PF₅⁻ or BARF⁻.

In certain embodiments, the disclosure relates to compositions comprising a diruthenium complex of the following formula, 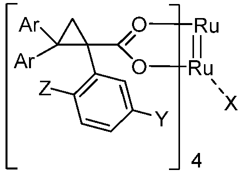
wherein X is a covalently bound ligand, such as a halogen, Cl, or a counterion such as PF₅⁻ or BARF⁻;
wherein Y is an electron withdrawing group; and
wherein Z is an electron withdrawing group.

In certain embodiments, Y is a halogen.

In certain embodiments, Z is a halogen.

In certain embodiments, the catalyst is in a composition with greater than 55%, 75%, 85%, 95%, 98%, or 99% enantiomeric excess of the following stereoisomers: 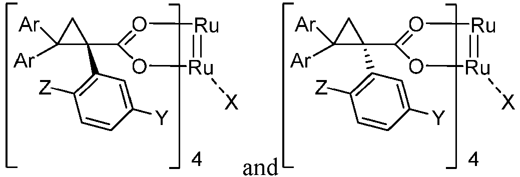
wherein X is a covalently bound ligand, such as a halogen, Cl, or a counterion such as PF₅⁻ or BARF⁻;
wherein Y is an electron withdrawing group; and
wherein Z is an electron withdrawing group.

In certain embodiments, Y is a halogen.

In certain embodiments, Z is a halogen.

In certain embodiments, the disclosure relates to compositions comprising a diruthenium complex of the following formula, 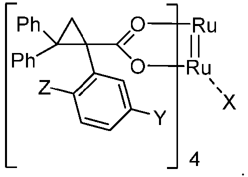
wherein X is a covalently bound ligand, such as a halogen, Cl, or a counterion such as PF₅⁻ or BARF⁻;
wherein Y is a halogen; and
wherein Z is a halogen.

In certain embodiments, the catalyst is in a composition with greater than 55%, 75%, 85%, 95%, 98%, or 99% enantiomeric excess of the following stereoisomers: 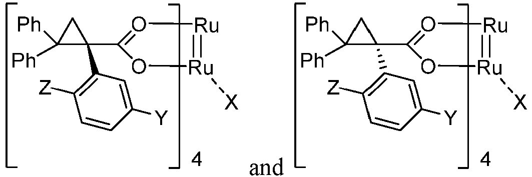
wherein X is a covalently bound ligand, such as a halogen, Cl, or a counterion such as PF₅⁻ or BARF⁻;⁻
wherein Y is a halogen; and
wherein Z is a halogen.

In certain embodiments, Y is a halogen.

In certain embodiments, Z is a halogen.

In certain embodiments, Y is a Br.

In certain embodiments, Z is a Cl.

In certain embodiments, the disclosure relates to a diruthenium catalyst comprising a pyrrolidine-2-sulfonamide substituted with a carboxylic acid ligand.

In certain embodiments, the disclosure relates to compositions comprising Ru₂(*R-*DOSP)₄ X , or a diruthenium complex of the following formula of formula 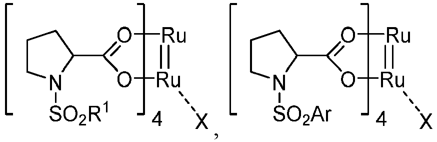 or derivatives or salts thereof wherein,
wherein X is a covalently bound ligand, such as a halogen, Cl, or a counterion such as PF₅⁻ or BARF⁻;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, Ar is p-alkyl-C₆H₄-.

In certain embodiments, this disclosure relates to compositions comprising a diruthenium complex of the following formula, 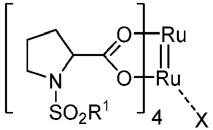
or salt thereof wherein,
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the counter anion is tetrakis(3,5-bis(trifluoromethyl)phenyl)borate (BArF).

In certain embodiments, the ligand is a halogen or chlorine (Cl).

In certain embodiments, R¹ is (p-alkylphenyl).

In certain embodiments, the diruthenium complex is tetrakis[N-(p-dodecylphenylsulfonyl)prolinato]diruthenium.

In certain embodiments, the disclosure relates to compositions comprising Ru₂(*S-*TCPTTL)₄ X , Ru₂(*S*-PTAD)₄ X, Ru₂(*S*-TCPTAD)₄ X or a diruthenium complex of the following formula of formula 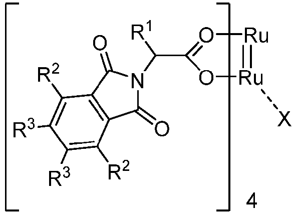
or derivatives or salts thereof wherein,
wherein X is a covalently bound ligand, such as a halogen, Cl, or a counterion such as PF₅⁻ or BARF⁻;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, R² is hydrogen.

In certain embodiments, R³ is hydrogen.

In certain embodiments, R² and R³ are hydrogen.

In certain embodiments, R¹ is tertbutyl.

In certain embodiments, R¹ is a carbocyclyl.

In certain embodiments, R¹ is adamantyl.

In certain embodiments, R² is phenyl.

In certain embodiments, R³ is phenyl.

In certain embodiments, R² and R³ are phenyl.

In certain embodiments, R² is halogen.

In certain embodiments, R³ is halogen.

In certain embodiments, R² and R³ are halogen.

In certain embodiments, this disclosure relates to composition comprising a diruthenium complex of the following formula, 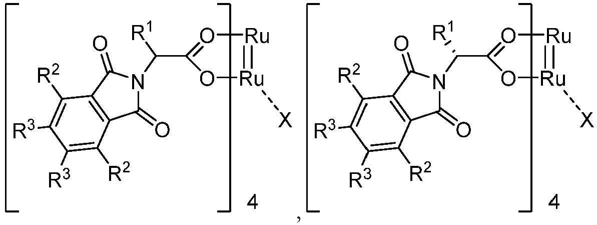 or 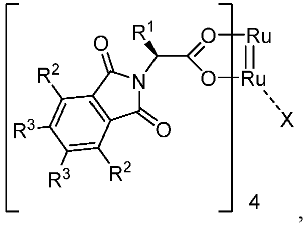
derivative or salt thereof wherein,
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the counter anion is tetrakis(3,5-bis(trifluoromethyl)phenyl)borate (BArF).

In certain embodiments, the ligand is a halogen or chlorine (Cl).

In certain embodiments, R¹ is a carbocyclyl or is adamantyl.

In certain embodiments, the diruthenium complex is tetrakis[2-(1,3-dioxo-4,5,6,7-tetraphenylisoindolin-2-yl)-3,3-dimethylbutanato]diruthenium.

In certain embodiments, the diruthenium complex is tetrakis[(1-adamantyl)-(N-tetrachlorophthalimido)acetato]diruthenium.

In certain embodiments, the diruthenium complex is tetrakis[(1-adamantyl)-(N-phthalimido)acetato] diruthenium.

### Synthetic Processes

Many enantioselective reactions of donor/acceptor carbenoids may be utilized with diruthenium catalysts disclosed herein. In certain embodiments, the diruthenium complex can be used to make intermediates for pharmaceutical agents as exemplified herein. See Davies & Manning, Nature, 2008, 451, 417-424; Davies & Denton, Chem. Soc. Rev., 2009, 38, 3061-3071; Davies, et al., J. Am. Chem. Soc., 2006, 128, 2485-2490; and Davies &. Walji, Angew. Chem., Int. Ed., 2005, 44, 1733-1735.

In certain embodiments, the disclosure contemplates reactions of donor/acceptor carbenoids, such as cyclopropanations, formal [4 + 3] cycloadditions, C-H functionalizations, and ylide transformations comprising mixing a compound comprising a carbenoid precursor, e.g., a diazo compound, and catalysts disclosed herein and reactive compounds under conditions such that a synthetic compound is formed.

In certain embodiments, the disclosure relates to methods of making a synthetic compound comprising mixing a) a diazo compound, b) a compound with a carbon hydrogen bond, and c) a diruthenium catalyst disclosed herein, under conditions such that a synthetic compound is formed comprising a carbon-to-carbon bond between the diazo compound and the compound with a carbon hydrogen bond. The synthetic compound may be the result of an inter or an intra-molecular reaction.

In certain embodiments, the disclosure relates to methods of making a synthetic compound comprising mixing a) a diazo compound, b) a compound with a nitrogen-to-hydrogen bond, and c) a diruthenium catalyst disclosed herein, under conditions such that a synthetic compound is formed comprising a carbon-to-nitrogen bond between the diazo compound and the compound with a nitrogen hydrogen bond. The synthetic compound may be the result of an inter or an intra-molecular reaction.

In certain embodiments, the disclosure relates to cyclopropanation reactions using catalysts disclosed herein. See Davies et al., J. Am. Chem. Soc. 1996, 118, 6897- 6907. In certain embodiments, the disclosure relates to methods of making a synthetic compound comprising mixing a) a diazo compound such as vinyldiazomethane and vinyldiazoactates optionally substituted with one or more substituents, b) a double bond compound such as an alkene or diene optionally substituted with one or more substituent, and c) a compound comprising a diruthenium catalyst disclosed herein under conditions such that a synthetic compound is formed comprising a cyclopropyl ring and carbon-to-carbon bond between the diazo compound and the double bond compound.

In certain embodiments, the diazo compound has the following formula, 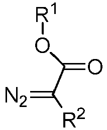 the double bond compound has the following formula, 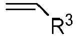 and the synthetic compound has the following formula, 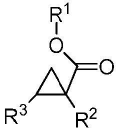
wherein, R¹ is alkyl, carbocyclyl, aryl, or heterocyclyl, wherein R¹ is optionally substituted with one or more, the same or different, R⁴;
R² is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, R¹ is alkyl optionally substituted with one or more, the same or different, R⁴.

In certain embodiments, R² is an electron donating group selected from cyano, alkenyl, alkynyl, formyl, carbamoyl, carboxy, alkylsulfinyl, alkylsulfonyl, or arylsulfonyl, aryl, or an aromatic heterocyclyl optionally substituted with one or more, the same or different, R⁴.

In certain embodiments, R² is aryl or an aromatic heterocyclyl optionally substituted with one or more, the same or different, R⁴.

In certain embodiments, R³ is an electron donating group selected from cyano, alkenyl, alkynyl, formyl, carbamoyl, carboxy, alkylsulfinyl, alkylsulfonyl, or arylsulfonyl, aryl, or an aromatic heterocyclyl optionally substituted with one or more, the same or different, R⁴.

In certain embodiments, R³ is aryl or an aromatic heterocyclyl optionally substituted with one or more, the same or different, R⁴.

In certain embodiments, the synthetic product is in a composition with greater than 55%, 75%, 85%, 95%, 98%, or 99% enantiomeric excess of the following stereoisomers:

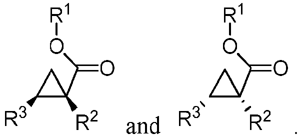

In certain embodiments, the diazo compound has the following formula, 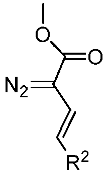 the double bond compound has the following formula, 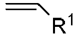 and the synthetic compound has the following formula, 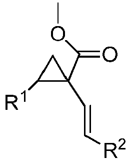
or salts thereof wherein
R¹ is phenyl optionally substituted with one or more substituent and
R² is phenyl optionally substituted with one or more substituent.

In certain embodiments, the synthetic product is in a composition with greater than 55%, 75%, 85%, 95%, 98%, or 99% enantiomeric excess of the following stereoisomers:

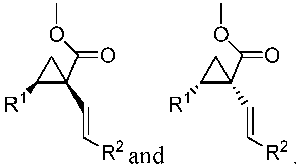

In certain embodiments, the diazo compound has the following formula, 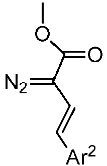 the double bond compound has the following formula, 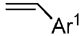 and the synthetic compound has the following formula, 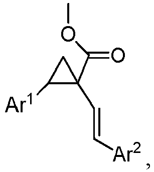 wherein Ar¹ and Ar² are each, the same or different, aryl optionally substituted with one or more substituents.

In certain embodiments, the synthetic product is in a composition with greater than 55%, 75%, 85%, 95%, 98%, or 99% enantiomeric excess of the following stereioisomers:

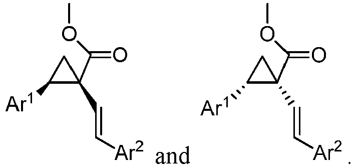

**In** certain embodiments, the disclosure contemplates the use of diruthenium catalysts disclosed herein in [4 + 3] cycloaddition reactions, e.g., mixing a diene and a vinyldiazoacetate and a diruthenium catalyst disclosed herein under conditions such that a cyclic compound is formed. See Davies et al, J. Am. Chem. Soc. 1998, 120, 3326- 3331 and Reddy & Davies, J. Am. Chem. Soc., 2007, 129 (34), 10312-10313.

In certain embodiments, the diazo compound is a vinyldiazoacetate of the following formula, 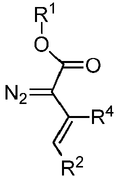 the diene compound has the following formula, 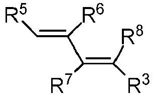 and the synthetic compound has the following formula, 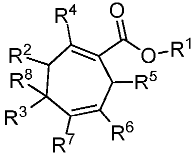
wherein, R¹ is alkyl, carbocyclyl, aryl, or heterocyclyl, wherein R¹ is optionally substituted with one or more, the same or different, R⁹;
R² is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁹;
R³ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁹;
R⁴ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁹;
R⁵ is hydrogen, alkyl, halogen, cyano, hydroxy, amino, mercapto, formyl, carboxy, carbamoyl, alkoxy, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁹;
R⁶ is hydrogen, alkyl, halogen, cyano, hydroxy, amino, mercapto, formyl, carboxy, carbamoyl, alkoxy, alkylthio, alkylamino, (alkyl)₂amino, alkylsulfinyl, alkylsulfonyl, arylsulfonyl, carbocyclyl, aryl, or heterocyclyl, wherein R⁶ is optionally substituted with one or more, the same or different, R⁹;
R⁷ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁷ is optionally substituted with one or more, the same or different, R⁹;
R⁸ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁸ is optionally substituted with one or more, the same or different, R⁹;
R⁹ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁹ is optionally substituted with one or more, the same or different, R¹⁰; and
R¹⁰ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the synthetic product is in a composition with greater than 55%, 75%, 85%, 95%, 98%, or 99% enantiomeric excess of the following stereoisomers:

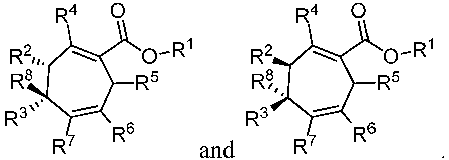

In certain embodiments, R¹ is alkyl optionally substituted with one or more, the same or different, R⁹.

In certain embodiments, R² is an electron donating group selected from cyano, alkenyl, alkynyl, formyl, carbamoyl, carboxy, alkylsulfinyl, alkylsulfonyl, or arylsulfonyl, aryl, or an aromatic heterocyclyl optionally substituted with one or more, the same or different, R⁹.

In certain embodiments, R² is aryl or an aromatic heterocyclyl optionally substituted with one or more, the same or different, R⁹.

In certain embodiments, R³ is an electron donating group selected from cyano, alkenyl, alkynyl, formyl, carbamoyl, carboxy, alkylsulfinyl, alkylsulfonyl, or arylsulfonyl, aryl, or an aromatic heterocyclyl optionally substituted with one or more, the same or different, R⁹.

In certain embodiments, R³ is aryl or an aromatic heterocyclyl optionally substituted with one or more, the same or different, R⁹.

In certain embodiments, R⁴ is hydrogen.

In certain embodiments, R⁵ is hydrogen.

In certain embodiments, R⁶ is hydrogen.

In certain embodiments, R⁷ is hydrogen.

In certain embodiments, R⁸ is hydrogen.

In certain embodiments, the diazo compound is a vinyldiazoacetate of the following formula, 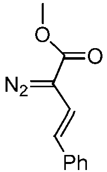 the diene compound has the following formula, 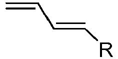 and the synthetic compound has the following formula,

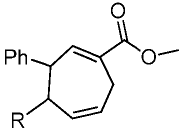

Wherein R is phenyl optionally substituted with one or more substituent.

In certain embodiments, the synthetic product is in a composition with greater than 55%, 75%, 85%, 95%, 98%, or 99% enantiomeric excess of the following stereioisomers:

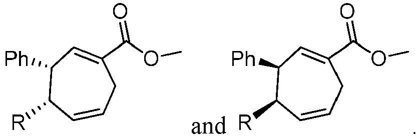

In certain embodiments, the diazo compound has the following formula, 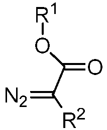 the compound has the following formula, 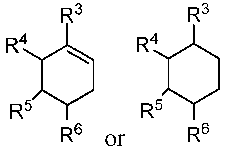 and the synthetic compound has the following formula, 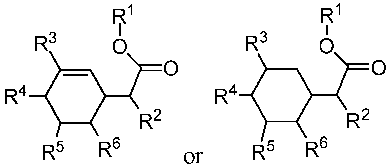
wherein, R¹ is alkyl, carbocyclyl, aryl, or heterocyclyl, wherein R¹ is optionally substituted with one or more, the same or different, R⁷;
R² is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁷;
R³ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁷;
R⁴ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁷;
R⁵ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁷; or R⁴ and R⁵ form a ring selected from a carbocyclyl, aryl, or heterocyclyl wherein the ring is optionally substituted with one or more, the same or different, R⁷
R⁶ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁶ is optionally substituted with one or more, the same or different, R⁷;
R⁷ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁷ is optionally substituted with one or more, the same or different, R⁸;
R⁸ is alkyl, halogen, cyano, hydroxy, amino, mercapto, formyl, carboxy, carbamoyl, alkoxy, alkylthio, alkylamino, (alkyl)₂amino, alkylsulfinyl, alkylsulfonyl, arylsulfonyl, carbocyclyl, aryl, or heterocyclyl, wherein R⁸ is optionally substituted with one or more, the same or different, R⁹; and
R⁹ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, R¹ is alkyl optionally substituted with one or more, the same or different, R⁷.

In certain embodiments, R² is an electron donating group selected from cyano, alkenyl, alkynyl, formyl, carbamoyl, carboxy, alkylsulfinyl, alkylsulfonyl, or arylsulfonyl, aryl, or an aromatic heterocyclyl optionally substituted with one or more, the same or different, R⁷.

In certain embodiments, R² is aryl or an aromatic heterocyclyl optionally substituted with one or more, the same or different, R⁷.

In certain embodiments, R³ is an electron donating group selected from cyano, alkenyl, alkynyl, formyl, carbamoyl, carboxy, alkylsulfinyl, alkylsulfonyl, or arylsulfonyl, aryl, or an aromatic heterocyclyl optionally substituted with one or more, the same or different, R⁷.

In certain embodiments, R³ is hydrogen or alkyl optionally substituted with one or more, the same or different, R⁷.

In certain embodiments, the disclosure relates to diazo compound of the following formula: 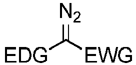 wherein EDG is an electron withdrawing group and EWG is an electron donating group. Such a diazo compound may react with a compound with a C-H bond or a N-H bond as further exemplified below. See Davies & Morton, Chem. Soc. Rev., 2011, 40, 1857-1869.

In certain embodiments, the diazo compound has the following formula, 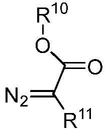
R¹⁰ is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹⁰ is optionally substituted with one or more, the same or different, R¹²;
R¹¹ is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹² is optionally substituted with one or more, the same or different, R⁹;
R¹² is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹² is optionally substituted with one or more, the same or different, R¹³;
R¹³ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, R¹⁰ is alkyl optionally substituted with one or more halogens.

In certain embodiments, R¹¹ is aryl or phenyl optionally substituted with an ortho or para substituted with one or more a halogen, hydroxy, alkoxy, thiol, alkylthio, amino, or alkylamino.

In certain embodiments, the method comprises mixing the diazo compound with a compound of the following formula: 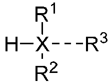 under conditions such that a synthetic compound of the following formula is formed, 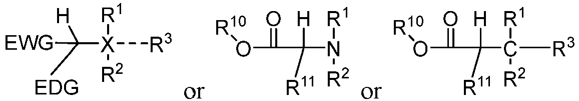
wherein X is carbon or nitrogen, wherein R³ is absent if X is nitrogen,
R¹ is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
or R¹ and R² form a carbocyclyl, aryl, or heterocyclyl ring optionally substituted with one or more, the same or different, R⁴; or when X is carbon R¹, R², and R³ form a multicyclic carbocyclyl;
R³ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl;
R¹⁰ is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹⁰ is optionally substituted with one or more, the same or different, R¹²;
R¹¹ is alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹¹ is optionally substituted with one or more, the same or different, R¹²;
R¹² is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹² is optionally substituted with one or more, the same or different, R¹³;
R¹³ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the disclosure contemplates the use of diruthenium catalysts disclosed herein for the intra-molecular insertion reactions for the formation of carbon-to-carbon bonds. In certain embodiment, disclosure contemplates a method of making a ring structure comprising mixing a diruthenium catalyst disclosed herein and a compound comprising a diazo group and the same compound comprising a C-H bond configure about the molecule to form a five or six membered ring.

In certain embodiments, the disclosure contemplates the use of diruthenium catalysts disclosed herein in C-H aminations. In certain embodiments, the disclosure contemplates the use of diruthenium catalysts disclosed herein for the insertion reactions for the formation of carbon to nitrogen bonds, e.g., inter and intra-molecular C-H aminations. See Reddy & Davies, Org. Lett., 2006, 8, 5013- 5016.

In certain embodiments, the disclosure relates to a method of making a synthetic compound comprising mixing a compound with an aromatic compound, PhI(OAc)₂, NsNH₂ (4-nitrobenzenesulfonamide , and a diruthenium catalysts disclosed herein under conditions such that a synthetic compound with a C-N bond is formed.

In certain embodiments, the aromatic compound is a has the following formula 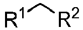 and the synthetic compound is 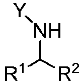
wherein, Y is 4-nitrobenzenesulfonamide or 2-nitrobenzenesulfonamide;
R¹ is aryl or aromatic heterocyclyl wherein R¹ is optionally substituted with one or more, the same or different, R³;
R² is hydrogen alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R³;
or R¹ and R² together with the attached atoms form a carbocyclyl, aryl, or heterocyclyl optionally substituted with one or more, the same or different, R³;
R³ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is hydrogen, alkyl, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

In certain embodiments, the synthetic product is in a composition with greater than 55%, 75%, 85%, 95%, 98%, or 99% enantiomeric excess of the following stereioisomers:

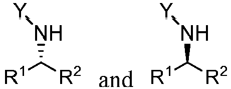

In certain embodiments, the disclosure relates to a method of making Rasagiline comprising the step of mixing 2,3-dihydro-1H-indene , PhI(OAc)₂, YNH₂, MgO, and a diruthenium catalyst disclosed herein, wherein Y is 4-nitrobenzenesulfonamide or 2-nitrobenzenesulfonamide, under conditions such that a compound of the following formula is formed,

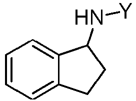

Reaction of this product with propargyl bromide in potassium carbonate provides the N substituted propargyl product. Removal of the nitrobenzenesulfonamide with DBU and HSCH₂CH₂OH provides Rasagiline. See Reddy & Davies, Org. Lett., 2006, 8, 5013-5016.

### EXPERIMENTAL

The following is intended to provide examples on methods of making and using embodiments of the disclosure. It is not intended to limit the scope as defined by the claims.

### Synthesis and characterization of a diruthenium catalysts

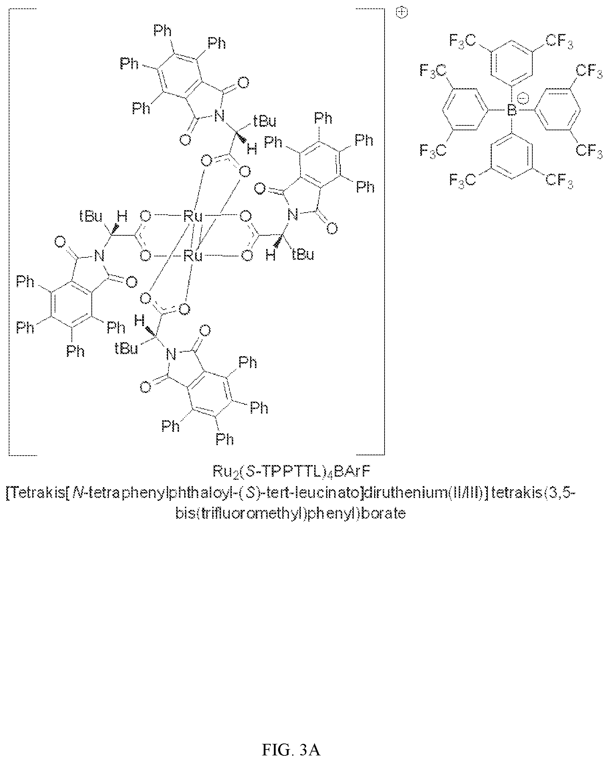

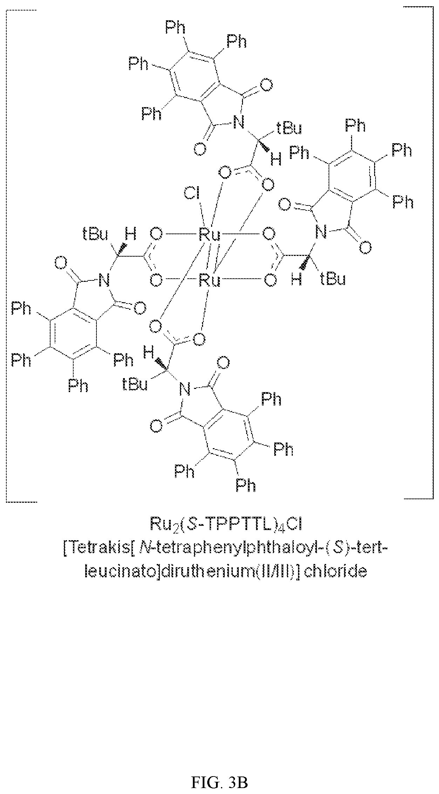

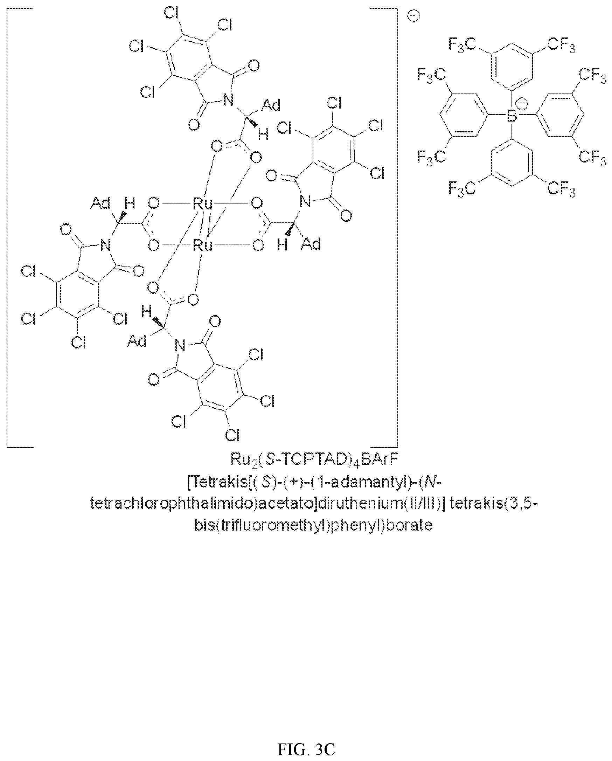

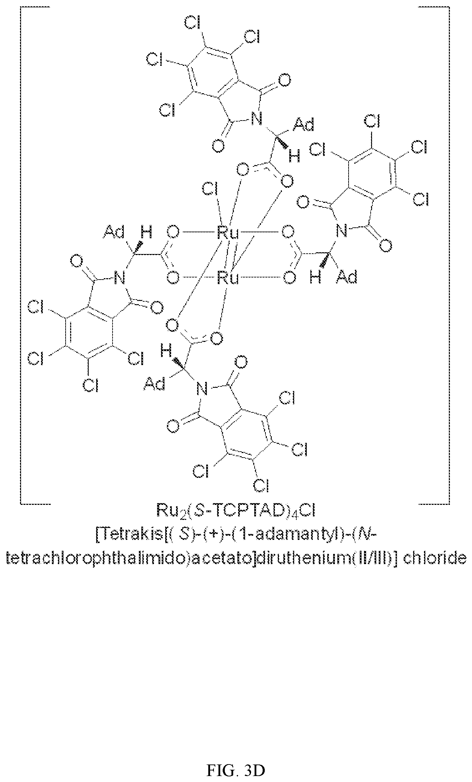

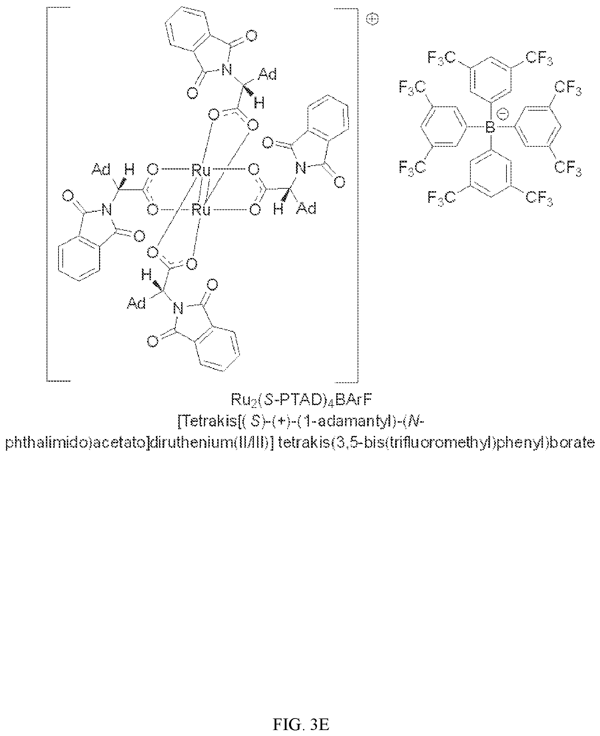

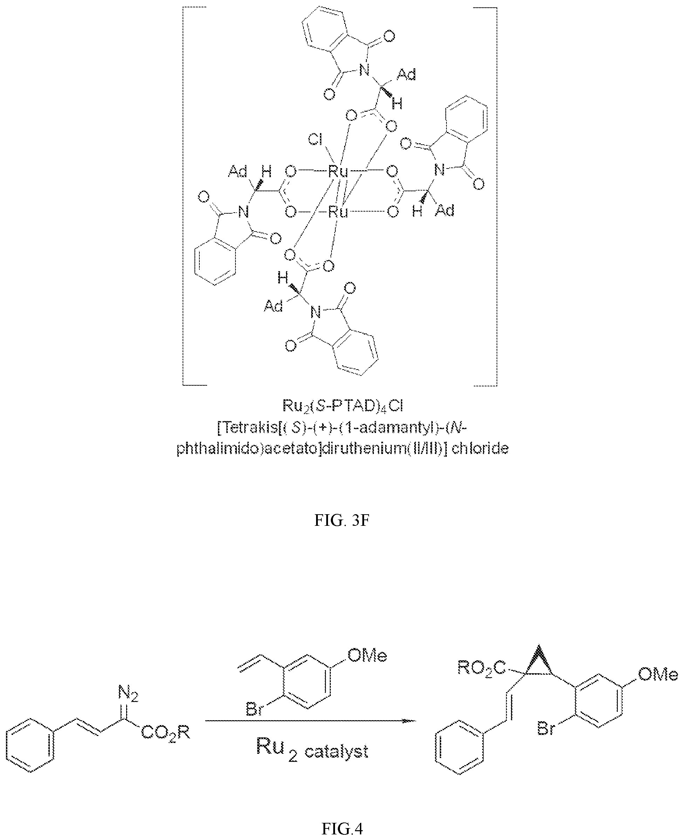

To confirm that the ligands self-assemble to generate the symmetry structures of diruthenium complexes studies were performed with a S-TPPTTL ligand (Figure 3B). Heating the ligand with Ru₂(OAc)₄Cl , results in the formation of a brown solid, that was confirmed by mass-spectrometry to be the desired diruthenium complex. Treatment of Ru₂(S-TPPTTL)₄Cl with NaBArF, displaces the ruthenium bound chloride and generates the diruthenium cationic species [Ru₂(S-TPPTTL)₄]⁺ BARF⁻. The X-ray structure of Ru₂(TPPTTL)₄Cl showed that the complex had adopted a C₄-symmetric bowl-shaped structure.

### Enantioselective cyclopropanation with donor/acceptor carbenes

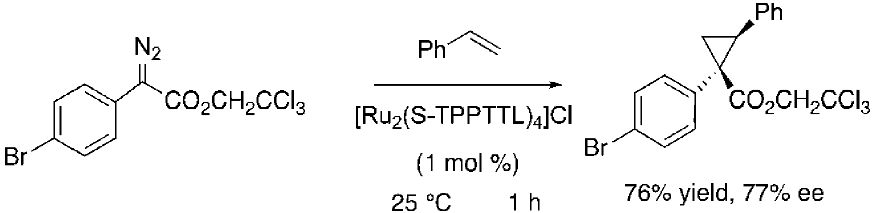

Results with diazomalonate gave moderate results (44% yield, 46% ee) because the reactions were conducted at 60 °C. However, aryldiazoacetates are more responsive to certain catalysts. As a test reaction a standard cyclopropanation reaction between an aryldiazoacetate and styrene was conducted. The reaction with Ru₂(S-TPPTTL)₄Cl was be conducted at room temperature and generated the cyclopropane in 76% yield and 77% ee.

### Enantioselective C-H functionalization

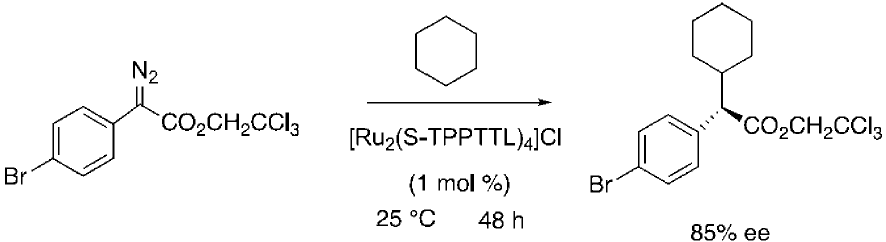

A reaction of the aryldiazoacetate with cyclohexane was performed. Cyclohexane is a challenging substate and because it is 28,000 times less reactive that styrene in certain reactions with donor/acceptor carbenes. It was observed that cyclohexane was an effective trap, generating the C-H functionalized product in 85% ee. Even more impressive is the reaction with [Ru₂(TPPTTL)₄]+ BArF- as catalyst at 25 °C, which generated the C-H functionalization product in 76% yield and 95% ee.

## Claims

1. A composition comprising a diruthenium complex of the following formula, 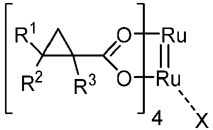
or salt thereof wherein,
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

2. The composition of claim 1 wherein the counter anion is tetrakis(3,5-bis(trifluoromethyl)phenyl)borate (BArF).

3. The composition of claim 1 wherein the ligand is a halogen or chlorine (Cl).

4. The composition of claim 1 wherein the diruthenium complex is
(i) tetrakis [1-(4-phenyl(phenyl))-2,2-diphenylcyclopropanecarboxylato]diruthenium;
(ii) tetrakis[1-(5-bromo-2-chlorophenyl)-2,2-diphenylcyclopropanecarboxylato] diruthenium;
(iii) tetrakis [1,2,2-tris[4'-(tert-butyl)-(1,1'-biphenyl]-4-yl]cyclopropane-1-carboxylato] diruthenium];
(iv) tetrakis [1-(4,4"-di-tert-butyl-[1,1':3',1"-terphenyl]-5'-yl)-2,2-diphenylcyclopropanecarboxylato] diruthenium; or
(v) tetrakis[1-(4-bromophenyl)-2,2-diphenylcyclopropanecarboxylato]diruthenium.

5. A composition comprising a diruthenium complex of the following formula, 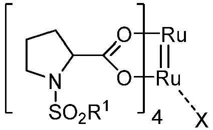
or salt thereof wherein,
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

6. The composition of claim 5 wherein the counter anion is tetrakis(3,5-bis(trifluoromethyl)phenyl)borate (BArF).

7. The composition of claim 5 wherein the ligand is a halogen or chlorine (Cl).

8. The composition of claim 5 wherein R¹ is (p-alkylphenyl).

9. The composition of claim 5 wherein the diruthenium complex is tetrakis[N-(p-dodecylphenylsulfonyl)prolinato]diruthenium.

10. A composition comprising a diruthenium complex of the following formula, 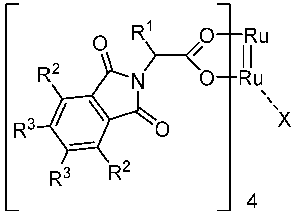
or salt thereof wherein,
X is a ligand or a counter anion;
R¹ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R¹ is optionally substituted with one or more, the same or different, R⁴;
R² is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R² is optionally substituted with one or more, the same or different, R⁴;
R³ is hydrogen, alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R³ is optionally substituted with one or more, the same or different, R⁴;
R⁴ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁴ is optionally substituted with one or more, the same or different, R⁵;
R⁵ is alkyl, halogen, cyano, hydroxy, amino, mercapto, formyl, carboxy, carbamoyl, alkoxy, alkanoyl, alkylthio, alkylamino, (alkyl)₂amino, alkylsulfinyl, alkylsulfonyl, arylsulfonyl, carbocyclyl, aryl, or heterocyclyl, wherein R⁵ is optionally substituted with one or more, the same or different, R⁶; and
R⁶ is halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, or heterocyclyl.

11. The composition of claim 10, wherein the counter anion is tetrakis(3,5-bis(trifluoromethyl)phenyl)borate (BArF).

12. The composition of claim 10, wherein the ligand is a halogen or chlorine (Cl).

13. The composition of claim 10, wherein R¹ is a carbocyclyl or is adamantyl.

14. The composition of claim 10, wherein the diruthenium complex is
(i) tetrakis[2-(1,3-dioxo-4,5,6,7-tetraphenylisoindolin-2-yl)-3,3-dimethylbutanato] diruthenium;
(ii) tetrakis[(1-adamantyl)-(N-tetrachlorophthalimido)acetato]diruthenium; or
(iii) tetrakis[(1-adamantyl)-(N-phthalimido)acetato] diruthenium.

15. A method of performing a synthetic transformation comprising contacting a diruthenium complex as in any of claims 1-14 with a starting material providing a coupling product.

## Patentansprüche

1. Zusammensetzung, umfassend einen Dirutheniumkomplex der folgenden Formel, 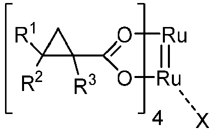
oder ein Salz davon, wobei
X für einen Liganden oder ein Gegenanion steht;
R¹ für Wasserstoff, Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R¹ gegebenenfalls durch ein oder mehrere gleiche oder verschiedene R⁴ substituiert ist;
R² für Wasserstoff, Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R² gegebenenfalls durch ein oder mehrere gleiche oder verschiedene R⁴ substituiert ist;
R³ für Wasserstoff, Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R³ gegebenenfalls durch ein oder mehrere gleiche oder verschiedene R⁴ substituiert ist;
R⁴ für Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R⁴ gegebenenfalls durch ein oder mehrere gleiche oder verschiedene R⁵ substituiert ist;
R⁵ für Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R⁵ gegebenenfalls durch ein oder mehrere gleiche oder verschiedene R⁶ substituiert ist; und
R⁶ für Halogen, Nitro, Cyano, Hydroxy, Trifluormethoxy, Trifluormethyl, Amino, Formyl, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, N-Methyl-N-ethylamino, Acetylamino, N-Methylcarbamoyl, N-Ethylcarbamoyl, N,N-Dimethylcarbamoyl, N,N-Diethylcarbamoyl, N-Methyl-N-ethylcarbamoyl, Methylthio, Ethylthio, Methylsulfinyl, Ethylsulfinyl, Mesyl, Ethylsulfonyl, Methoxycarbonyl, Ethoxycarbonyl, N-Methylsulfamoyl, N-Ethylsulfamoyl, N,N-Dimethylsulfamoyl, N,N-Diethylsulfamoyl, N-Methyl-N-ethylsulfamoyl, Carbocyclyl, Aryl oder Heterocyclyl steht.

2. Zusammensetzung nach Anspruch 1, wobei es sich bei dem Gegenanion um Tetrakis(3,5-bis(trifluormethyl)phenyl)borat (BArF) handelt.

3. Zusammensetzung nach Anspruch 1, wobei es sich bei dem Liganden um ein Halogen oder Chlor (Cl) handelt.

4. Zusammensetzung nach Anspruch 1, wobei es sich bei dem Dirutheniumkomplex um Folgendes handelt:
(i) Tetrakis[1-(4-phenyl(phenyl))-2,2-diphenylcyclopropancarboxylato]diruthenium;
(ii) Tetrakis[1-(5-brom-2-chlorphenyl)-2,2-diphenylcyclopropancarboxylato]diruthenium;
(iii) Tetrakis[1,2,2-Tris[4'-(tert-butyl)-(1,1'-biphenyl]-4-yl]cyclopropan-1-carboxylato]diruthenium];
(iv) Tetrakis[1-(4,4"-di-tert-butyl-[1,1':3',1"-terphenyl]-5'-yl)-2,2-diphenylcyclopropancarboxylato]diruthenium; oder
(v) Tetrakis[1-(4-bromphenyl)-2,2-diphenylcyclopropancarboxylato]diruthenium.

5. Zusammensetzung, umfassend einen Dirutheniumkomplex der folgenden Formel, 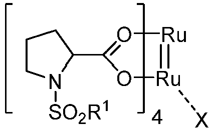
oder ein Salz davon, wobei
X für einen Liganden oder ein Gegenanion steht;
R¹ für Wasserstoff, Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R¹ gegebenenfalls durch ein oder mehrere gleiche oder verschiedene R⁴ substituiert ist;
R⁴ für Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R⁴ gegebenenfalls durch ein oder mehrere gleiche oder verschiedene R⁵ substituiert ist;
R⁵ für Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R⁵ gegebenenfalls durch ein oder mehrere gleiche oder verschiedene R⁶ substituiert ist; und
R⁶ für Halogen, Nitro, Cyano, Hydroxy, Trifluormethoxy, Trifluormethyl, Amino, Formyl, Carboxy, Carbamoyl,
Mercapto, Sulfamoyl, Methyl, Ethyl, Methoxy, Ethoxy, Acetyl, Acetoxy, Methylamino, Ethylamino, Dimethylamino, Diethylamino, N-Methyl-N-ethylamino, Acetylamino, N-Methylcarbamoyl, N-Ethylcarbamoyl, N,N-Dimethylcarbamoyl, N,N-Diethylcarbamoyl, N-Methyl-N-ethylcarbamoyl, Methylthio, Ethylthio, Methylsulfinyl, Ethylsulfinyl, Mesyl, Ethylsulfonyl, Methoxycarbonyl, Ethoxycarbonyl, N-Methylsulfamoyl, N-Ethylsulfamoyl, N,N-Dimethylsulfamoyl, N,N-Diethylsulfamoyl, N-Methyl-N-ethylsulfamoyl, Carbocyclyl, Aryl oder Heterocyclyl steht.

6. Zusammensetzung nach Anspruch 5, wobei es sich bei dem Gegenanion um Tetrakis(3,5-bis(trifluormethyl)phenyl)borat (BArF) handelt.

7. Zusammensetzung nach Anspruch 5, wobei es sich bei dem Liganden um ein Halogen oder Chlor (Cl) handelt.

8. Zusammensetzung nach Anspruch 5, wobei R¹ für (p-Alkylphenyl) steht.

9. Zusammensetzung nach Anspruch 5 , wobei es sich bei dem Dirutheniumkomplex um Tetrakis[N-(p-dodecylphenylsulfonyl)prolinato]diruthenium handelt.

10. Zusammensetzung, umfassend einen Dirutheniumkomplex der folgenden Formel, 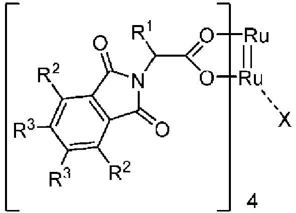
oder ein Salz davon, wobei
X für einen Liganden oder ein Gegenanion steht;
R¹ für Wasserstoff, Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R¹ gegebenenfalls durch ein oder mehrere gleiche oder verschiedene R⁴ substituiert ist;
R² für Wasserstoff, Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R² gegebenenfalls durch ein oder mehrere gleiche oder verschiedene R⁴ substituiert ist;
R³ für Wasserstoff, Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R³ gegebenenfalls durch ein oder mehrere gleiche oder verschiedene R⁴ substituiert ist;
R⁴ für Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R⁴ gegebenenfalls durch ein oder mehrere gleiche oder verschiedene R⁵ substituiert ist;
R⁵ für Alkyl, Halogen, Cyano, Hydroxy, Amino, Mercapto, Formyl, Carboxy, Carbamoyl, Alkoxy, Alkanoyl, Alkylthio, Alkylamino, (Alkyl)₂amino, Alkylsulfinyl, Alkylsulfonyl, Arylsulfonyl, Carbocyclyl, Aryl oder Heterocyclyl steht, wobei R⁵ gegebenenfalls durch ein oder mehrere gleiche oder verschiedene R⁶ substituiert ist; und
R⁶ für Halogen, Nitro, Cyano, Hydroxy, Trifluormethoxy, Trifluormethyl, Amino, Formyl, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, N-Methyl-N-ethylamino, Acetylamino, N-Methylcarbamoyl, N-Ethylcarbamoyl, N,N-Dimethylcarbamoyl, N,N-Diethylcarbamoyl, N-Methyl-N-ethylcarbamoyl, Methylthio, Ethylthio, Methylsulfinyl, Ethylsulfinyl, Mesyl, Ethylsulfonyl, Methoxycarbonyl, Ethoxycarbonyl, N-Methylsulfamoyl, N-Ethylsulfamoyl, N,N-Dimethylsulfamoyl, N,N-Diethylsulfamoyl, N-Methyl-N-ethylsulfamoyl, Carbocyclyl, Aryl oder Heterocyclyl steht.

11. Zusammensetzung nach Anspruch 10, wobei es sich bei dem Gegenanion um Tetrakis(3,5-bis(trifluormethyl)phenyl)borat (BArF) handelt.

12. Zusammensetzung nach Anspruch 10, wobei es sich bei dem Liganden um ein Halogen oder Chlor (Cl) handelt.

13. Zusammensetzung nach Anspruch 10, wobei R¹ für ein Carbocyclyl oder für Adamantyl steht.

14. Zusammensetzung nach Anspruch 10, wobei es sich bei dem Dirutheniumkomplex um Folgendes handelt:
(i) Tetrakis[2-(1,3-dioxo-4,5,6,7-tetraphenylisoindolin-2-yl)-3,3-dimethylbutanato]diruthenium;
(ii) Tetrakis[(1-adamantyl)-(N-tetrachlorphthalimido)acetato]diruthenium; oder
(iii) Tetrakis[(1-adamantyl)-(N-phthalimido)acetato]diruthenium.

15. Verfahren zur Durchführung einer synthetischen Transformation, umfassend das Inkontaktbringen eines Dirutheniumkomplexes wie in einem der Ansprüche 1-14 mit einem Ausgangsstoff, was ein Kupplungsprodukt liefert.

## Revendications

1. Composition comprenant un complexe de diruthénium de la formule suivante, 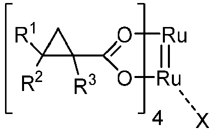
ou un sel correspondant, dans laquelle,
X est un ligand ou un contre-anion ;
R¹ est hydrogène, alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R¹ est éventuellement substitué par un ou plusieurs, identiques ou différents, R⁴ ;
R² est hydrogène, alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R² est éventuellement substitué par un ou plusieurs, identiques ou différents, R⁴ ;
R³ est hydrogène, alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R³ est éventuellement substitué par un ou plusieurs, identiques ou différents, R⁴ ;
R⁴ est alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R⁴ est éventuellement substitué par un ou plusieurs, identiques ou différents, R⁵ ;
R⁵ est alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R⁵ est éventuellement substitué par un ou plusieurs, identiques ou différents, R⁶ ; et
R⁶ est halogène, nitro, cyano, hydroxy, trifluorométhoxy, trifluorométhyle, amino, formyle, carboxy, carbamoyle, mercapto, sulfamoyle, méthyle, éthyle, méthoxy, éthoxy, acétyle, acétoxy, méthylamino, éthylamino, diméthylamino, diéthylamino, N-méthyl-N-éthylamino, acétylamino, N-méthylcarbamoyle, N-éthylcarbamoyle, N,N-diméthylcarbamoyle, N,N-diéthylcarbamoyle, N-méthyl-N-éthylcarbamoyle, méthylthio, éthylthio, méthylsulfinyle, éthylsulfinyle, mesyle, éthylsulfonyle, méthoxycarbonyle, éthoxycarbonyle, N-méthylsulfamoyle, N-éthylsulfamoyle, N,N-diméthylsulfamoyle, N,N-diéthylsulfamoyle, N-méthyl-N-éthylsulfamoyle, carbocyclyle, aryle, ou hétérocyclyle.

2. Composition selon la revendication 1, dans laquelle le contre-anion est le tétrakis(3,5-bis(trifluorométhyl)phényl)borate (BArF).

3. Composition selon la revendication 1, dans laquelle le ligand est un halogène ou chlore (Cl).

4. Composition selon la revendication 1, dans laquelle le complexe de diruthénium est
(i) tétrakis[1-(4-phényl(phényl))-2,2-diphénylcyclopropanecarboxylato]diruthénium ;
(ii) tétrakis[1-(5-bromo-2-chlorophényl)-2,2-diphénylcyclopropanecarboxylato]diruthénium ;
(iii) tétrakis[1,2,2-tris[4'-(tert-butyl)-(1,1'-biphényl]-4-yl]cyclopropane-1-carboxylato]diruthénium] ;
(iv) tétrakis[1-(4,4"-di-tert-butyl-[1,1':3',1"-terphényl]-5'-yl)-2,2-diphénylcyclopropanecarboxylato]diruthénium ; ou
(v) tétrakis[1-(4-bromophényl)-2,2-diphénylcyclopropanecarboxylato]diruthénium.

5. Composition comprenant un complexe de diruthénium de la formule suivante, 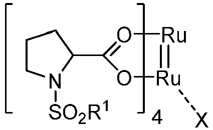
ou un sel correspondant, dans laquelle,
X est un ligand ou un contre-anion ;
R¹ est hydrogène, alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R¹ est éventuellement substitué par un ou plusieurs, identiques ou différents, R⁴ ;
R⁴ est alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R⁴ est éventuellement substitué par un ou plusieurs, identiques ou différents, R⁵ ;
R⁵ est alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R⁵ est éventuellement substitué par un ou plusieurs, identiques ou différents, R⁶ ; et
R⁶ est halogène, nitro, cyano, hydroxy, trifluorométhoxy, trifluorométhyle, amino, formyle, carboxy, carbamoyle, mercapto, sulfamoyle, méthyle, éthyle, méthoxy, éthoxy, acétyle, acétoxy, méthylamino, éthylamino, diméthylamino, diéthylamino, N-méthyl-N-éthylamino, acétylamino, N-méthylcarbamoyle, N-éthylcarbamoyle, N,N-diméthylcarbamoyle, N,N-diéthylcarbamoyle, N-méthyl-N-éthylcarbamoyle, méthylthio, éthylthio, méthylsulfinyle, éthylsulfinyle, mesyle, éthylsulfonyle, méthoxycarbonyle, éthoxycarbonyle, N-méthylsulfamoyle, N-éthylsulfamoyle, N,N-diméthylsulfamoyle, N,N-diéthylsulfamoyle, N-méthyl-N-éthylsulfamoyle, carbocyclyle, aryle, ou hétérocyclyle.

6. Composition selon la revendication 5, dans laquelle le contre-anion est le tétrakis(3,5-bis(trifluorométhyl)phényl)borate (BArF).

7. Composition selon la revendication 5, dans laquelle le ligand est un halogène ou chlore (Cl).

8. Composition selon la revendication 5, dans laquelle R¹ est (p-alkylphényle).

9. Composition selon la revendication 5, dans laquelle le complexe de diruthénium est le tétrakis[N-(p-dodécylphénylsulfonyl)prolinato]diruthénium.

10. Composition comprenant un complexe de diruthénium de la formule suivante, 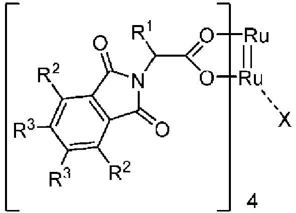
ou un sel correspondant, dans laquelle,
X est un ligand ou un contre-anion ;
R¹ est hydrogène, alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R¹ est éventuellement substitué par un ou plusieurs, identiques ou différents, R⁴ ;
R² est hydrogène, alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R² est éventuellement substitué par un ou plusieurs, identiques ou différents, R⁴ ;
R³ est hydrogène, alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R³ est éventuellement substitué par un ou plusieurs, identiques ou différents, R⁴ ;
R⁴ est alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R⁴ est éventuellement substitué par un ou plusieurs, identiques ou différents, R⁵ ;
R⁵ est alkyle, halogène, cyano, hydroxy, amino, mercapto, formyle, carboxy, carbamoyle, alcoxy, alcanoyle, alkylthio, alkylamino, (alkyl)₂amino, alkylsulfinyle, alkylsulfonyle, arylsulfonyle, carbocyclyle, aryle, ou hétérocyclyle, dans laquelle R⁵ est éventuellement substitué par un ou plusieurs, identiques ou différents, R⁶ ; et
R⁶ est halogène, nitro, cyano, hydroxy, trifluorométhoxy, trifluorométhyle, amino, formyle, carboxy, carbamoyle, mercapto, sulfamoyle, méthyle, éthyle, méthoxy, éthoxy, acétyle, acétoxy, méthylamino, éthylamino, diméthylamino, diéthylamino, N-méthyl-N-éthylamino, acétylamino, N-méthylcarbamoyle, N-éthylcarbamoyle, N,N-diméthylcarbamoyle, N,N-diéthylcarbamoyle, N-méthyl-N-éthylcarbamoyle, méthylthio, éthylthio, méthylsulfinyle, éthylsulfinyle, mesyle, éthylsulfonyle, méthoxycarbonyle, éthoxycarbonyle, N-méthylsulfamoyle, N-éthylsulfamoyle, N,N-diméthylsulfamoyle, N,N-diéthylsulfamoyle, N-méthyl-N-éthylsulfamoyle, carbocyclyle, aryle, ou hétérocyclyle.

11. Composition selon la revendication 10, dans laquelle le contre-anion est le tétrakis(3,5-bis(trifluorométhyl)phényl)borate (BArF).

12. Composition selon la revendication 10, dans laquelle le ligand est un halogène ou chlore (Cl).

13. Composition selon la revendication 10, dans laquelle R¹ est un carbocyclyle ou est adamantyle.

14. Composition selon la revendication 10, dans laquelle le complexe de diruthénium est
(i) tétrakis[2-(1,3-dioxo-4,5,6,7-tetraphénylisoindolin-2-yl)-3,3-diméthylbutanato]diruthénium ;
(ii) tétrakis[(1-adamantyl)-(N-tétrachlorophtalimido)acétato]diruthénium ; ou
(iii) tétrakis[(1-adamantyl)-(N-phtalimido)acétato]diruthénium.

15. Procédé de réalisation d'une transformation synthétique comprenant la mise en contact d'un complexe de diruthénium selon l'une quelconque des revendications 1-14 avec une matière de départ fournissant un produit de couplage.
